# NEW EUROPEAN PATENT SPECIFICATION

(11) **EP 3 021 833 B2**
(45) Date of publication and mention of the opposition decision: **30.03.2022**
(45) Mention of the grant of the patent: 27.06.2018
(21) Application number: 14744505.0
(22) Date of filing: 21.07.2014
(51) Int. Cl.: A61K 9/00, A61K 47/12, C07K 16/24

(54) **METHODS AND FORMULATIONS WHICH ALLOW THE MODULATION OF IMMUNE RESPONSES RELATED TO THE ADMINISTRATION OF A BIOPHARMACEUTICAL DRUG**
VERFAHREN UND FORMULIERUNGEN ZUR ERMÖGLICHUNG DER MODULATION VON IMMUNREAKTIONEN IN ZUSAMMENHANG MIT DER VERABREICHUNG EINES BIOPHARMAZEUTISCHEN ARZNEIMITTELS
PROCÉDÉS ET FORMULATIONS QUI PERMETTENT À LA MODULATION DE RÉPONSES IMMUNITAIRES ASSOCIÉES À L'ADMINISTRATION D'UN MÉDICAMENT BIOPHARMACEUTIQUE

(30) Priority: 19.07.2013 EP 13177259
(43) Date of publication of application: 25.05.2016
(73) Proprietor: Hexal Aktiengesellschaft, 83607 Holzkirchen (DE)
(72) Inventor: KRONTHALER, Ulrich, 83607 Holzkirchen (DE); VIERTLBÖCK-SCHUDY, Margot, 82041 Oberhaching (DE); BARON, Melanie, 82041 Oberhaching (DE)
(74) Representative: Michalski Hüttermann & Partner Patentanwälte mbB
(86) International application number: PCT/EP2014/065612
(87) International publication number: WO 2015/007912

(56) References cited:
- WO-A1-2012/089778
- WO-A2-2013/011076
- US-A1- 2012 237 547
- FATTOM A ET AL: "Comparative Immunogenicity of Conjugates Composed of the Staphylococcus aureus Type 8 Capsular Polysaccharide Bound to Carrier Proteins by Adipic Acid Dihydrazide or N-Succinimidyl-3-(2-Pyridyldithio)propiona te", INFECTION AND IMMUNITY, AMERICAN SOCIETY FOR MICROBIOLOGY, vol. 60, no. 2, 1 February 1992 (1992-02-01), pages 584-589, XP002103339, ISSN: 0019-9567

## Description

### Field of the Invention

The present invention relates to the use of hexanedioic acid, or at least one salt thereof, in the absence of citric acid for reducing the immunogenicity of a pharmaceutical formulation comprising an anti-TNFα antibody.

### Background to the Invention

For multiple reasons, such as molecule size, many modem drugs such as biopharmaceuticals are not suitable for oral administration. Instead they need to be injected invasively either into the extravascular space, e.g. subcutaneously (s.c.), intramuscularly (i.m.), or directly into the vascular system, *e.g.* intravenously (i.v.). From these options, the subcutaneous route is typically most preferred. One of the main reasons is that it allows self-administration by patients, which is of special importance in the case of chronic therapies. In addition, is it less invasive as compared to intravenous administration and, accordingly, associated risks are generally lower, as in case of unintentionally injecting at low hygienic conditions. Subcutaneous administration is favourable although resorption from the subcutaneous space is only partial, and low bioavailability generally requires an increase of dose which translates to higher costs for therapy.

In addition to its functions of regulation and sensation, the primary function of skin is protection. It acts as a barrier against mechanical stress but also shields the individual as a first line barrier against micro-organisms, as is illustrated by an increased risk of infection/inflammation in case the skin is lesioned. As the mechanical functionality alone would be insufficient, pH and for some sites, proteases, serve as additional chemical defence mechanisms against pathogens. Importantly, one highly efficient mechanism of protection is the increased surveillance of the tissue by the immune system. While physiologically detection of pathogens is desired, processing of biopharmaceuticals by the immune system like pathogens is in most cases unwanted. The increased surveillance thus explains the second key downside of subcutaneous administration of biopharmaceuticals, namely unwanted immunogenicity, in addition to increased costs arising through higher amounts of the product but also higher efforts in manufacturing and development of this product class. Such unwanted immunogenicity may result in numerous consequences. It may lead to loss of efficacy, increased clearance from circulation or trigger potentially life threatening anaphylactic reactions. As loss of efficacy may be noticed after a long period, which may also mean unnecessary costs to the health care system, due to the high price of this product class. While most preferable from a patient and medical care taker perspective, limited bioavailability in combination with risks such as immunogenicity pose a serious problem.

Since patient related risk factors for occurrence of immunogenicity, such as genetic disposition, are poorly understood and accordingly difficult to control, controlling and optimising product related risk factors are the only opportunity to allow patients to fully benefit from modem therapies, such as biologics, especially since they are prescribed only in case all other therapeutic options have already failed, also as a consequence of their high costs.

Desired modulation in this context may in most cases mean aiming to decrease immunogenicity, since in the majority of cases, drugs having the potential to be recognised by the immune system, including subsequent formation of antibodies against, are only fully efficacious and safe when no such immune response occurs. On the other hand, therapies exist which specifically aim to cause a suitable immune response in order to eliminate or inactivate the intended target. The respective target could be a foreign protein that should be recognised/neutralised.

In all these cases it might be desirable to further increase the intended immune response resulting from the drug, and in such cases the presence of citric acid in the administration solution or at preceding manufacturing stages might be beneficial in this respect.

Formulations of the state of the art, for example Humira^{®} used as a formulation of the anti-TNF-alpha (anti-TNFα) antibody adalimumab comprising phosphate as well as citric acid as a buffer substance, meet the requirements as to tolerability upon administration, stability of the biopharmaceutical drug and shelf life.

However, said formulations have a number of disadvantages. One disadvantage is that many biopharmaceuticals upon subcutaneous administration, induce unwanted immunogenicity as outlined above. Due to their antigenic properties, other protein-like contaminations, which are difficult to detect analytically, can also trigger immunological reactions in humans. Moreover, proteins of animal origin can trigger immunological reactions in humans due to their species-specific properties in general. Even if the risk for such reactions is already low for state of the art manufacturing methods, it might accumulate over time, in particular upon chronic reapplication of such drugs.

This is, in particular, to be feared in case of subcutaneous application, as removal and distribution via the bloodstream is much more delayed in comparison to intravenous application. A favourable formulation would thus contribute to reducing drug induced immunogenicity and providing a more cost effective medicament, or to increasing drug induced immunogenicity, in case this is the intended reaction as a prophylactic or therapeutic intervention.

WO 2012089778 discloses pharmaceutical formulations comprising a biopharmaceutical drug, said compositions further comprising at least one mono- or dicarboxylic acid with a backbone of 2-6 C-Atoms, or at least one salt thereof, and a method to prepare the formulation. The formulation may further comprise an additional acid/salt, a stabilizer which can be an amino acid, a sugar polyol, a disaccharide and/or a polysaccharide.

The problem underlying the present invention is to provide a use of formulation components which does not show the above described disadvantages of the hitherto known dosage forms, aiming at a low immunogenicity. It should, in particular, be suitable for use in selfapplication by patients or trained medical personal and it should be characterised by the capability to reduce immunogenicity and/or at the same time avoid undesired irritations of the skin and/or pain at the injection site often occurring along with the (self-) application of a drug by means of an injection.

Thus, there is a need to provide suitable pharmaceutical formulations for parental, in particular subcutaneous, administration/injection.

This problem is solved by the embodiments characterised in the claims. The use according to the present invention offers advantages in the preparation of formulations to reduce immunogenicity of biopharmaceutical drugs as described in the present description. The invention is based on the surprising observation that the immune response towards anti-TNFα can be modulated by selecting an appropriate formulation comprising hexanedioic acid, or at least one salt thereof. This renders the formulations of the present invention suitable and advantageous for the use in ready-to-use syringes and kits provided therewith, such as for home use.

### Summary of the invention

It is to be understood that the terminology used herein is for purposes of describing particular embodiments only, and is not intended to be limiting. It must be noted that, as used in the specification and the appended claims, the singular forms "a," "an", and "the" include singular and/or plural referents unless the context clearly dictates otherwise. It is moreover to be understood that, in case parameter ranges are given which are delimited by numeric values, the ranges are deemed to include these limitation values.

The present invention relates to the use of hexanedioic acid, or at least one salt thereof, in the absence of citric acid for reducing the immunogenicity of a pharmaceutical formulation comprising an anti-TNFα antibody.

The present invention further relates to said use, wherein said use comprises dissolving said anti-TNFα antibody in a buffer comprising at least hexanedioic acid or at least one salt thereof.

The present invention further relates to said use, wherein the formulation further comprises at least one stabiliser selected from the group consisting of an amino acid, a sugar polyol, a disaccharide and/or a polysaccharide.

The present invention further relates to said use, wherein said disaccharide is at least one agent selected from the group consisting of sucrose, trehalose, maltose and/or lactose.

The present invention further relates to said use, wherein said sugar polyol is at least one agent selected from the group consisting of mannitol and/or sorbitol.

The present invention further relates to said use, wherein the formulation is designed to be administered intramuscularly or subcutaneously.

The present invention further relates to said use, wherein the formulation is designed to be administered non-orally.

Hexanedioic acid is a carboxylic acid compound, which is also called adipic acid, 1,4-butanedicarboxylic acid, 1,6-hexanedioic acid, acifloctin, acinetten, adipinic acid, octafluorohexanedioic acid or molten adipic acid. Adipic acid has the molecular formula of C₆H₁₀O₄ and a molecular weight of 146.1 g/mol. The terms "adipic acid" and "hexanedioic acid" are used interchangeably herein.

According to an aspect of the invention, the use of hexanedioic acid, or at least one salt thereof, in the absence of citric acid for reducing the immunogenicity of a pharmaceutical formulation comprising an anti-TNFα antibody is provided, which use allows to reduce the immunogenicity of a pharmaceutical formulation comprising an anti-TNFα antibody.

In accordance with the present invention, the term "pharmaceutical formulation" relates to any formulation which is suitable to be administered to a patient and comprises a drug which exhibits a therapeutic and/or diagnostic effect. Such "pharmaceutical formulation" is, preferably, a drug delivery formulation.

As used herein, the term "immunogenicity" and/or "immunogenic" and/or "rate of immunogenicity" shall be understood in terms of the potential of a molecule, when injected into a subject, to be recognised by the immune system, including subsequent reactions, be they intended or unintended such as formation of antibodies against the molecule, *i.e.* could cause/induce a response affecting the immune system of the subject.

As used in this disclosure, the term "modulation of immune responses" shall be understood as either increasing or reducing the administration-related immunogenicity of a given formulation. While in many cases, a reduction of immunogenicity may be desirable in order to increase patients' compliance and reduce harmful side effects, an increase of immunogenicity may be desirable in other cases.

The term "reduced immunogenicity" as used herein refers to a formulation designed to comprise at least one biopharmaceutical molecule which, when injected into a patient, e.g. subcutaneously, will cause/induce an immune response which is not substantially stronger than an immune response induced by a reference molecule denoted as being essentially not immunogenic.

As used herein, the term "biopharmaceutical drug" shall include any therapeutic or diagnostic molecule being derived from a biological or from a biotechnological source, or chemically synthesised to be equivalent to a product from said source, for example, a protein, a peptide, a nucleic acid, an immunoglobulin, a polysaccharide, a cell product, a plant extract, an animal extract, a recombinant protein or combinations thereof. Commonly, the biopharmaceutical drug is the active ingredient of a biopharmaceutical. The terms "drug" and "compound" are used interchangeably herein and are intended to mean (an) agent(s) and/or medicine(s) used to diagnose, prevent, or treat the symptoms a disease, physical or mental condition, injury or infection.

The term "bioavailability" refers to the degree to which or rate at which a drug or other substance is absorbed or becomes available at the site of physiological activity after administration. The bioavailability of a macromolecule may be assayed by *in vivo* pharmacokinetics methods known in the art.

Suitable assays for the assessment of immunogenicity are well known in the art and comprise qualitative and semi-quantitative binding assays, neutralising assays, and confirmatory assays (immuno- as well as immunochromatographic-assays) for the measurement of anti-drug antibodies (ADAs). Further assays comprise qualitative assays for total antibody determination (screen and titre), confirmatory assay (specificity) by use of ELISA, ECL, RIA and flow cytometry formats (including acid dissociation "AD" and solid phase extraction "SPEAD" and "BEAD" assays for increased drug tolerance). Cell-based assays for the detection of neutralising antibodies and toxicokinetics analysis for large molecules are also well known.

The terms "subject" or "individual" or "animal" or "patient" or "mammal", mean any subject, particularly a mammalian subject, for whom diagnosis, prognosis, prevention, or therapy is desired. A "mammal", for purposes of treatment, refers to any animal classified as a mammal, including but not limited to humans, domestic and farm animals, and zoo, sports, or pet animals, such as dogs, horses, cats, cows, and the like. Preferably, the mammal is human.

In the process of experiments conducted within the scope of the present disclosure, advantageous properties of hexanedioic acid-buffered formulations were surprisingly found to comprise a good acceptability/compatibility in rabbits upon subcutaneous injection and a lower clearance in comparison to commercially available drugs and reference formulations after one week after dosing. Thus, as shown in Examples 1 and 2, the immunogenicity can be reduced by selecting an appropriate concentration of hexanedioic acid in the formulation or application solution. This can be specifically desirable for drugs, with the potential to be recognised by the immune system.

Many factors influence the immunogenicity of pharmaceutical formulations comprising biopharmaceutical drugs, including structural features (sequence variation and glycosylation), storage conditions (denaturation or aggregation caused by oxidation), contaminants or impurities in the preparation, dose and length of treatment, as well as the route of administration, appropriate formulation and the genetic characteristics of patients. The clinical manifestations have been outlined in the section above.

As outlined in detail in the experimental section, the inventors surprisingly found that a formulation comprising hexanedioic acid features a reduced immune response upon subcutaneous injection in rabbits compared to the injection of formulations comprising citric acid. At the same time, the inventors surprisingly found that a formulation which has a reduced content, or is essentially free, of citric acid, or a salt thereof, features a reduced immune response upon subcutaneous injection in rabbits. The latter is particularly striking because, generally, small molecules like organic acids do not evoke immune responses at all. Without being bound to theory, the inventors postulate that citric acid, or is salts, may either be immunogenic as such, or play a role in the development of an immune response.

Thus, it is prudent to expect that formulating a pharmaceutical drug in a hexanedioic acid buffer will induce less irritation or immune response compared to, for example, citric acid buffers. At the same time, it is prudent to expect that formulating a pharmaceutical drug in a buffer which has a reduced content, or is essentially free, of citric acid, or ist salts, will induce less irritation or immune response compared to, for example, citric acid buffers. The present disclosure discloses these surprising observations for the first time, which, applied accordingly, allow to affect, reduce, minimise and/or modulate immunogenicity of drugs that are recognised by the immune system through formulation, without impacting bioavailability.

The use of the present disclosure can be altered as would be known to the skilled artisan, but generally would follow a procedure such as described hereinafter. First, a an anti-TNFα antibody is obtained in a desired concentration in a solvent such as water, preferably supplemented with further suitable buffer expedients such as NaCl, Tween 80 or other buffer expedients well known in the art at pH values between 5 to 8, wherein the an anti-TNFα antibody is stable. The pharmaceutical formulation according to the present invention contains the an anti-TNFα antibody in an amount that is effective for achieving a therapeutic effect.

In the second step, said biopharmaceutical drug is dissolved in a buffer comprising at least hexanedioic acid or at least one salt thereof in a suitable concentration as described in detail below to allow the formation of a formulation or a suspension of the biopharmaceutical drug. In the preparation of the liquid formulation according to the present invention, the buffer substance is preferably provided in form of its free acid. The desired pH value of the solution is adjusted by the addition of bases, like for example alkali hydroxides, earth alkali hydroxides or ammonium hydroxide. For this purpose, the use of sodium hydroxide is preferred.

Usually, a formulation for a biopharmaceutical drug comprises one or more buffer(s), isotonising agent(s) and water for injection as a solvent. Additionally, stabilisers are frequently added, like for example a cryoprotective agent. Furthermore, one or more metal chelating agent(s) and tenside(s) can be added. Some agents may have a double role, *e.g.*, some sugars or sugar alcohols can serve as a cryoprotective agent and isotonising agent.

As a skilled person will appreciate, one or more further suitable acid(s) can be added to produce a formulation in suitable amounts. Particularly preferred are mono- or dicarboxylic acids, or a salt thereof, which at least one selected from the group consisting of:
- acetic acid, or acetate
- glutamic acid, or glutamate
- malic acid, or malate
-
- phosphoric acid, and/or phosphate
- tartaric acid, or tartrate, and/or
- succinic acid, or succinate.

Certain acid(s) known to be recognised by the immune system, *i.e.* being immunogenic, and/or to induce an immune response in a subject/patient are not regarded as being suitable for the preparation of a formulation for reducing immunogenicity according to the present invention.

Further additives such as stabilisers, surfactans, isotonising agents, metal ion chelators, etc. can be added to the formulation and/or suspension, which additives are suitable for the desired application and are well known to a person skilled in the art. As used herein, the term "stabiliser" shall refer to an agent which helps to maintain the structural integrity of the biopharmaceutical drug, particularly during freezing and/or lyophilization and/or storage.

Such agents are, in the context of the present invention, also called "cryoprotectant" or "lyoprotectant".

As an optional step of the use according to the present invention, the obtained formulation and/or suspension can be lyophilised by using standard techniques which are well known to the skilled person. In a preferred embodiment of the invention, said formulation is in a form selected from the group consisting of
a) aqueous form
b) lyophilised form, and/or
c) suspension.

In aqueous form, said formulation may be ready for administration, while in lyophilised form said formulation can be transferred into liquid form prior to administration, e.g., by addition of water for injection which may or may not comprise a preservative such as benzyl alcohol, antioxidants like vitamin A, vitamin E, vitamin C, retinylpalmitate, and selenium, the amino acids cysteine and methionine, citric acid and sodium citrate, synthetic preservatives like the parabens methyl paraben and propyl paraben.

Finally, the obtained formulation, suspension and/or the lyophilised formulation can be processed for example by adding preservatives for long term storage or filled in a suitable receptacle for the desired administration. Hence, the formulation according to the use of the present invention is useful for the parental administration of a therapeutic and/or diagnostic drug. The parental administration can be intramuscular or subcutaneous, wherein the subcutaneous application is particularly preferred.

Particularly, the formulation of the present disclosureis designed for as well as non-oral delivery routes, such as - but not limited to - parenteral administration, topical administration, pulmonary delivery.

Thus, in a preferred embodiment the present invention relates to the use of hexanedioic acid, or at least one salt thereof, in the absence of citric acid for reducing the immunogenicity of a pharmaceutical formulation comprising an anti-TNFα antibody as mentioned above and described in detail below. Preferably, the formulation is in a non-oral dosage form, i.e. designed to be administered non-orally.

Preferably, hexanedioic acid is used as the only carboxylic acid or salt, or even as the only main buffer in the formulation according to the present invention.

Particularly preferred, the formulation may be substantially free of at least one further buffer compound selected from the group consisting of:
- acetic acid, or acetate
- glutamic acid, or glutamate
- malic acid, or malate
- phosphoric acid and/or phosphate
- tartaric acid, or tartrate, and/or
- succinic acid, or succinate.
or any other expedient for which is known to induce or increase immunogenicity upon injection into a subject.

According to the present disclosure, it is provided that the use results in a reduction of immune responses related to the administration thereof..

The term "reduced content of citric acid", as used herein, means that the formulation does preferably not contain more than 0.01, 0.05, 0.1 0.2, 0.3, 0.4, 0.5 0.6 0.7 0.8 0.9 1.0, 1.2 or 1.3 mM citric acid, or a salt thereof.

This concentration range can also encompass cumulative concentrations of citric acid, and a salt thereof, e.g., sodium citrate. In case citric acid is provided as a hydrate (e.g., monohydrate), the respective concentrations should be corrected accordingly.

The term "essentially free of citric acid", as used herein, means that no citric acid, or salt thereof, has intentionally been be added to the composition. The total amount of citric acid, or salts thereof as a product of unintended contamination is therefore below 0.05%, preferably below 0.01%. Most preferred is a composition in which no amount citric acid, or a salt thereof, can be detected with standard analytical methods used in pharmaceutical technology.

It is particularly preferred that hexanedioic acid used in the use according to the present invention, in a concentration of between ≥ 1 and ≤ 100 mM, preferably between ≥ 2 and ≤ 50 mM, and even more preferably between ≥ 5 and ≤ 25 mM, most preferably 23 mM. It is to be understood that every numerical value between 1 mM to 100 mM can be used in accordance with the present invention and depends on the intended use of the formulation and the patient to be treated.

It is furthermore preferred that, in addition to hexanedioic acid, the pharmaceutical formulation may also contain phosphate, preferably sodium phosphate (herein also called "NaP"). The term "sodium phosphate" is to be understood as to embrace sodium dihydrogen phosphate and disodium hydrogenphosphate, and all conceivable salts and/or hydrates thereof.

Said aqueous form of the pharmaceutical formulation has, preferably, a pH of between ≥ 3 and ≤ 9, preferably between ≥ 4 and ≤ 8, more preferably between ≥ 5 and ≤ 7.

In yet another preferred embodiment of the present invention, the use further comprises at least one stabiliser selected from the group consisting of an amino acid, a sugar polyol, a disaccharide and/or a polysaccharide.

Preferably, said disaccharide is at least one agent selected from the group consisting of sucrose, trehalose, maltose and/or lactose.

Likewise preferably, said sugar polyol is at least one agent selected from the group consisting of mannitol and/or sorbitol. Among these, mannitol is a particularly preferred sugar polyol. Preferably, it is used as the only sugar polyol, or even the only stabiliser in the pharmaceutical formulation according to the present invention.

It is particularly preferred that said stabiliser is present in an aqueous form of the pharmaceutical formulation in a concentration of between ≥ 1 mM and ≤ 300 mM, preferably between ≥ 2 mM and ≤ 200 mM, and more preferably between ≥ 5 mM and ≤ 150 mM.

In yet another preferred embodiment of the present invention, said formulation further comprises at least one agent selected from the group consisting of:
- a surfactant
- an isotonising agent, and/or
- a metal ion chelator.

Said surfactant enhances the wetability of the components and supports their solubility. This is particularly important because biopharmaceutical drugs are often formulated in high concentrations (e.g., > 100 mg in 1 - 10 ml).

Suitable surfactants are for example lecithin and other non-ionic tensides, like polysorbates ("Tween"), or poloxameres. Particularly preferred tensides are polysorbate 80 ("Tween 80") or poloxamere 188.

Said isotonising agent serves for setting the osmotic pressure of the formulation according to the invention to a physiologically acceptable value, *e.g.* to the osmolarity of blood.

The isotonising agent is a physiologically acceptable compound and is not particularly limited. Typical examples of the isotonising agent are, for instance, an inorganic salt such as sodium chloride, potassium chloride or calcium chloride, and the like. These can be used alone or in a mixture thereof.

Said metal ion chelator serves for complex formation of heavy metals, which otherwise may inactivate the biopharmaceutical drug comprised in the formulation according to the invention. Preferably said metal ion chelator is EDTA and/or EGTA.

While there is increasing evidence that high amounts of citric acid may have an impact on immunogenicity when subcutaneously injected, which immunogenicity is likely to induce and/or increase the clearance of a pharmaceutical drug contained in a citric acid buffered composition, it is desired to avoid an unwanted clearance for pharmaceutical drugs which need to be present in an active form in the body.

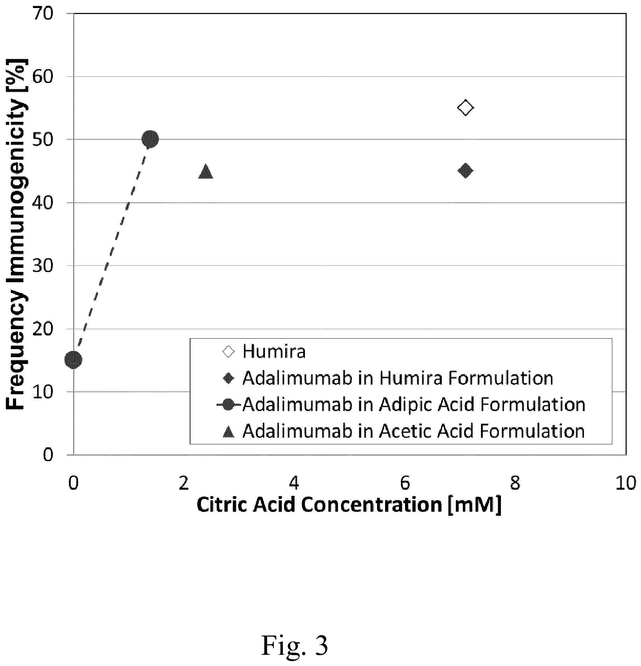

As outlined in Example 2 as well as Fig. 3 and Table 4, the use of the adipic acid-buffered adalimumab containing formulation without citric acid surprisingly exhibits a negliably low immune response when injected subcutaneously. As a result, a formulation buffered by adipic acid results in a very low level of immune response, and thus renders this formulation particularly superior for subcutaneous injection.

Thus, in yet another preferred embodiment of said use, said formulation is a formulation suitable for parenteral administration, preferably for intramuscular and/or subcutaneous administration. It is particularly preferred that the formulation is designed for the subcutaneous administration.

In a preferred embodiment of the present invention, said use is suitable to maintain the structural integrity of an anti-TNFα antibody stored therein in terms of aggregation, protection potential and stability such as thermodynamic stability.

The following table shows formulations comprising a chimeric, humanised or human antibody (IgG format) which show superior aggregation behaviour over control buffers comprising a phosphate/citrate system, as for example disclosed in WO2012/089778 submitted by the assignee, content of which is herewith expressly incorporated by reference.

| **Compound** | **mol weight** | **[mg/mL]** | **Example concentration (mM)** | **Example concentration (mg/mL)** |
|---|---|---|---|---|
| chimeric, humanised or human IgG | | 5 - 100.00 | | 50 mg/mL |
| NaCl | 58.4 | 5.0 - 7.0 | 105.00 | 6.132 |
| Mannitol | 182 | 10.0 - 14.0 | 66.00 | 12.012 |
| Adipic acid | 146.14 | 1.5 - 4.0 | 23.00 | 3.36122 |
| Citric acid | 192.124 | 0 - 9.6 | 1.30 | 0.2497612 |
| Tween 80 | 1.310 | 0.5 - 1.5 | 0.80 | 1.048 |
| NaOH (to adjust pH) pH | | | q.s. 5.20 | |

Analytical techniques for measuring protein stability are available in the art and are reviewed *e.g.* in Peptide and Protein Drug Delivery, 247-301 (Vincent Lee ed., New York, N.Y., 1991) and Jones, 1993 Adv. Drug Delivery Rev. 10: 29-90. Stability can be measured at a selected temperature for a selected time period as exemplified by the provided examples. Storage of stable formulations (*i.e.* shelf life) is preferably for at least 6 months, more preferably 12 months, more preferably 12-18 months, and more preferably for 2 or more years.

According to the present disclosure, said biopharmaceutical drug is a protein. Said protein can be a naturally occurring protein, a modified protein (*i.e.*, a protein which has been modified with respect to its natural counterpart, also termed scaffold, or template) or a fully synthetic protein (*i.e.*, a protein which has no natural counterpart).

Said protein can either be isolated from a natural organism, or it can be obtained by fermentation of a cultured organism. Furthermore, said protein can be a protein which is either a homologue or a heterologue to the protein which has been obtained from the organism. Furthermore, said protein can be a recombinant protein.

According to the present disclosure, the biopharmaceutical drug is an immunoglobulin. Preferably, it is an IgG.

It is preferred that the anti-TNFα antibody is present in an aqueous form of the pharmaceutical formulation in a concentration of between ≥ 0.1 and ≤ 500 mg ml⁻¹, preferably between ≥ 20 and ≤ 200 mg ml⁻¹.

The term "immunoglobulin" is meant to include, but is not limited to, an antibody and antibody fragment (such as scFv, Fab, Fc, F(ab')2), and other genetically engineered portions of antibodies. Depending on the amino acid sequence of the constant domain of their heavy chains, immunoglobulins can be assigned to different classes. There are five major classes of immunoglobulins: IgA, IgD, IgE, IgG and IgM. Several of these may be further divided into subclasses (isotypes), *e.g.*, IgG1, IgG2, IgG3, and IgG4; IgA1 and IgA2. The heavy chain constant domains that correspond to the different classes of immunoglobulins are called alpha (α) delta (δ), epsilon (ε), gamma (γ), and mu (µ), respectively. The subunit structures and three-dimensional configurations of different classes of immunoglobulins are well known.

Particularly preferred in accordance with the present invention, said antibody, or fragment or derivative thereof, selected from the group consisting of:
(i) hybridoma-derived antibody
(ii) chimerised antibody
(iii) humanised antibody, and/or
(iv) human antibody.

It is particularly preferred that said immunoglobulin is a monoclonal antibody, or a fragment or derivative thereof.

As used herein, the term "monoclonal antibody (mAb)", shall refer to an antibody composition having a homogenous antibody population, *i.e.*, a homogeneous population consisting of a whole immunoglobulin, or a fragment or derivative thereof Particularly preferred, such antibody is selected from the group consisting of IgG, IgD, IgE, IgA and/or IgM, or a fragment or derivative thereof.

As used herein, the term "fragment" shall refer to fragments of such antibody retaining, in some cases, target binding capacities, e.g.
- a CDR (complementarity determining region)
- a hypervariable region,
- a variable domain (Fv)
- an IgG heavy chain (consisting of VH, CH1, hinge, CH2 and CH3 regions)
- an IgG light chain (consisting of VL and CL regions), and/or
- a Fab and/or F(ab)₂.

As used herein, the term "derivative" shall refer to protein and/or immunoglobulin constructs being structurally different from, but still having some structural relationship to, the common antibody concept, e.g., scFv, Fab and/or F(ab)₂, as well as bi-, tri- or higher specific antibody constructs. All these items are explained below.

Other antibody derivatives known to the skilled person are diabodies, camelid antibodies, domain antibodies, bivalent homodimers with two chains consisting of scFvs, IgAs (two IgG structures joined by a J chain and a secretory component), shark antibodies, antibodies consisting of new world primate framework plus non-new world primate CDR, dimerised constructs comprising CH3+VL+VH, and antibody conjugates (*e.g*. antibody or fragments or derivatives linked to a toxin, a cytokine, a radioisotope or a label).

Methods for the production and/or selection of chimeric, humanised and/or human mAbs are known in the art. For example, US6331415 by Genentech describes the production of chimeric antibodies, while US6548640 by Medical Research Council describes CDR grafting techniques and US5859205 by Celltech describes the production of humanised antibodies. In vitro antibody libraries are, among others, disclosed in US6300064 by MorphoSys and US6248516 by MRC/Scripps/Stratagene. Phage Display techniques are for example disclosed in US5223409 by Dyax. Transgenic mammal platforms are for example described in US200302048621 by TaconicArtemis.

IgG, scFv, Fab and/or F(ab)₂ are antibody formats well known to the skilled person. Related enabling techniques are available from the respective textbooks.

As used herein, the term "Fab" relates to an IgG fragment comprising the antigen binding region, said fragment being composed of one constant and one variable domain from each heavy and light chain of the antibody.

As used herein, the term "F(ab)₂" relates to an IgG fragment consisting of two Fab fragments connected to one another by disulfide bonds.

As used herein, the term "scFv" relates to a single-chain variable fragment being a fusion of the variable regions of the heavy and light chains of immunoglobulins, linked together with a short linker, usually serine (S) or glycine (G). This chimeric molecule retains the specificity of the original immunoglobulin, despite removal of the constant regions and the introduction of a linker peptide.

Modified antibody formats are for example bi- or trispecific antibody constructs, antibody-based fusion proteins, immunoconjugates and the like.

In the present invention, said antibody, or fragment or derivative thereof, is an anti-TNF-α-antibody.

One example for an anti-TNF-α-antibody is defined by the sequence listing enclosed in the international application WO2012089778. Therein, SEQ ID No 1 defines the encoding nucleic acid sequence of the IgG heavy chain, SEQ ID No 2 defines the encoding nucleic acid sequence of the IgG light chain, and SEQ ID Nos 3 and 4 define the amino acid sequences of the heavy chain and the light chain, respectively

SEQ ID Nos 5, 7 and 9 define the amino acid sequences of the complementarity determining regions (CDR) of the light chain (*i.e.*, LC CDR 3, LC CDR 2 and LC CDR 1). SEQ ID Nos 6, 8 and 10 define the amino acid sequences of the complementarity determining regions of the heavy chain (*i.e.*, HC CDR 3, HC CDR 2 and HC CDR 1).

Note that the SEQ ID Nos 1 and 2, or parts thereof, can by equivalently replaced by
- nucleic acid sequences encoding for the same proteins, or the protein chains, encoded by SEQ ID Nos 1 and 2, but have nucleotide substitutions which are tolerable under the degeneracy of the genetic code,
- sequences encoding a fraction, variant, homologue, or derivative of the proteins, or the protein chains, encoded by SEQ ID Nos 1 and 2,
- nucleic acid sequences which are code optimised for a given expression host, and/or
- nucleic acid molecule having a sequence identity of at least 70, preferably 95 % with either SEQ ID No 1 or 2.

In a particularly preferred embodiment, the pharmaceutical formulation used comprises an antibody that comprises (i) an amino acid sequence that is at least 80% identical to the amino acid sequence of SEQ ID No 1 and/or SEQ ID No 2; and/or (ii) CDR regions that have amino acid sequences that are identical to the amino acid sequences of the CDR regions of adalimumab.

In one embodiment, the antibody of the invention comprises at least one heavy or light chain CDR of an antibody molecule known as adalimumab. In another embodiment, an antibody of the invention comprises at least two CDRs from one or more antibody molecules. In another embodiment, an antibody of the invention comprises at least three CDRs from one or more antibody molecules. In another embodiment, an antibody of the invention comprises at least four CDRs from one or more antibody molecules. In another embodiment, an antibody of the invention comprises at least five CDRs from one or more antibody molecules. In another embodiment, an antibody of the invention comprises at least six CDRs from one or more antibody molecules.

In another embodiment, the pharmaceutical formulation or the formulation obtainable by the method according to the present invention comprises an antibody that comprises an amino acid sequence that is at least 80%, 85%, 90% or 95% identical to the amino acid sequence of SEQ ID No 1 and/or SEQ ID No 2. In particular, said antibody comprises, consists essentially of, or consists of an immunoglobulin light and/or heavy chain variable region (V_{L} and/or V_{H}), where at least one of the V_{L}-CDRs of the light chain variable region or at least two of the V_{L}-CDRs of the light chain variable region are at least 80%, 85%, 90% or 95% identical to reference light chain V_{L}-CDR1, V_{L}-CDR2 or V_{L}-CDR3 and/or V_{H}-CDR4, V_{H}-CDR5 or V_{H}-CDR6 amino acid sequences from antibody as described by the SEQ ID Nos 1 to 10.

Note that the SEQ ID Nos 3 to 10, or parts thereof, can be equivalently replaced by amino acid sequences carrying one or more conservative amino acid substitution(s), *i.e.*, one or more substitution(s) which do not affect significant protein features, like target binding affinity, immunogenicity, ADCC response, serum half-life, solubility and so forth.

Alternatively, said antibody is an antibody mimetic, *i.e.*, a non-immunoglobulin-based targetbinding protein molecule. Many of the above mentioned techniques are applicable for these molecules as well. Such antibody mimetics are for example derived from Ankyrin Repeat Proteins, C-Type Lectins, A-domain proteins of *Staphylococcus aureus*, transferrins, lipocalins, fibronectins, Kunitz domain protease inhibitors, ubiquitin, cysteine knots or knottins, thioredoxin A, and so forth, and are known to the skilled person in the art from the respective literature.

In another alternative, said antibody is a recombinant fusion protein comprising any of the above mentioned proteins or substantially similar proteins. For example, recombinant fusion proteins comprising one of the above-mentioned proteins plus a multimerisation domain, such as a leucine zipper, a coiled coil, an Fc portion of an antibody, or a substantially similar protein, can be a biopharmaceutical drug comprised by the formulations of the present invention. Specifically included among such recombinant fusion proteins are proteins in which at least a portion of TNFR or RANK is fused to an Fc portion of an antibody. Particularly preferred, said recombinant fusion proteins comprise a target binding domain and the IgG Fc domain (so-called -cept molecules).

Since for example certain antibodies must be subcutaneously applied on a regular basis, such as daily, once a week, biweekly (*i.e.*, every 13-15 days) and/or once a month and certain buffers currently used for such a purpose can induce upon injection into the skin local irritations and/or pain and/or induce clearance of the injected antibody, it is desired to provide a method which is effective in modulating such as preventing, minimising and/or reducing immunogenicity.

As a skilled person will appreciate, certain acids used as main buffer compounds, such as citric acid, suffer from the drawback that they - upon subcutaneous injection - induce skin irritation as well as pain around the injection side. Surprisingly, the present disclosure reveals for the first time, as shown in the Examples, that citric acid in concentrations ranging from 1.3 mM to 9.6 mM strongly influences the immunogenicity upon subcutaneous injection of a protein such as the antibody adalimumab.

In one embodiment, the buffered composition according to the use of the present invention is intended for the preparation of a medicament, preferably for subcutaneous or intramuscular administration, for example by injection with prefilled syringes or by infusion, in case a long-term continuous administration of a biopharmaceutical drug as defied above is desirable.

As already mentioned above and illustrated in the Examples of WO2012/089778, the hexanedioic buffered formulation of adalimumab is stable over a long period of time and can basically be stored in any suitable receptacle. Accordingly, the present invention also relates to a receptacle containing one of the formulations described above and/or in the Examples.

Typically, the receptacle is a container that is conventionally intended for the storage and/or administration of a biopharmaceutical drug, like a vial, a syringe, an injection pen, an ampoule, a carpoule, or an infusion container, wherein the formulation according to the present invention is particularly advantageous for the use in ready-to-use syringes, pens and ampoules. In a preferred embodiment, the liquid formulation is present in the syringe or in the pen at an efficacious concentration, which is - in terms of an anti-TNF-alpha antibody such as adalimumab, 40 mg in 0.8 ml.

In one embodiment, the present invention relates to a prefilled syringe or pen, a vial or an infusion bag, said syringe or pen, vial or infusion bag comprising a pharmaceutical formulation or the formulations/suspension and/or lyophilised form obtainable by the method according to the present invention.

In still another aspect of the use of the present invention, a primary packaging, such as a prefilled syringe or pen, a vial, or an infusion bag is provided, comprising the formulation of the present invention.

The prefilled syringe or pen may contain the formulation either in lyophilised form (which has then to be solubilised, e.g., with water for injection, prior to administration), or in aqueous form. Said syringe or pen is often a disposable article for single use only and may have a volume between 0.1 and 20 ml. However, the syringe or pen may also be a multi-use or multidose syringe or pen.

Said vial may also contain the formulation in lyophilised form or in aqueous form and may serve as a single or multiple use device. As a multiple use device, said vial can have a bigger volume.

Said infusion bag usually contains the formulation in aqueous form and may have a volume between 20 and 5,000 ml.

In a further aspect of the use according to the present invention, the pharmaceutical formulation or the formulation/suspension and/or lyophilised formulation obtainable by the method and/or said primary packaging as mentioned above is for use in the treatment of at least one pathologic condition selected from the group consisting of:
- autoimmune diseases
- infectious diseases
- neoplastic and/or malignant diseases (cancer), and
- diseases of the nervous system.

Suitable autoimmune diseases are arthritic and rheumatic diseases, like psoriasis, morbus Crohn (Crohn's disease) or rheumatoid arthritis. Suitable infectious diseases are viral and/or bacterial infections. Suitable neoplastic and/or malignant diseases are sarcomas, carcinomas, lymphomas and leukaemias, preferably, lung cancer, breast cancer, ovarian cancer, colon cancer, prostate cancer, cervical cancer and the like. Suitable diseases of the nervous system are, among others, neurodegenerative disorders like Parkinson's disease, Alzheimer's disease, multiple sclerosis, Huntington's disease, or amyotrophic lateral sclerosis.

In other respects and with the same dosage strength, the formulations comprising an anti-TNF-alpha antibody according to the present invention can be used according to the product information of Humira^{®} (INN: adalimumab), in particular with regard to dosage, administration and medical indication.

Since admixing further excipients or taking further preparatory measures, like filtrating, mixing, etc., prior to the administration of the formulation of biopharmaceutical drug according to the present invention can advantageously be omitted, the biopharmaceutical drug according to the present invention can be prepared for immediate administration, for example in a kit.

In an embodiment that is particularly advantageous for physicians, pharmacists and especially for patients, the present disclosure therefore also relates to a kit for the parenteral administration of the formulation obtainable by the method of the present invention or the pharmaceutical formulation useful in the method of reducing immunogenicity, comprising one or more of the above-described receptacles, preferably along with instructions for storage and/or administration.

Preferably, 1 or 2 or 3 or 4 or 5 syringes or pens are provided in the kit according to the present disclosure, optionally more, like 7 syringes or pens, for example in case the daily administration is intended to last for one week.

For reasons of safe handling, the kit according to the present disclosure advantageously has safety compartments for syringes and for injection and/or infusion needles, respectively. Here, discharge aids for the needles and prepared or pre-fitted sealing caps are also to be considered.

As is described above, the formulations according to the use according to the present invention, the present invention are stable over a long period of time, in particular at about 5°C, preferably over a period of at least 4 weeks. Therefore, the formulations, receptacles and kits according to the present invention can advantageously be stored in a conventional refrigerator.

These and further embodiments resulting from the present invention are encompassed by the claims.

The disclosure of the prior art documents cited above and in the following is herewith incorporated in the present application by reference, in particular with respect to the production of an anti-TNF antibody and the hexanedioic acid buffer. These and further embodiments are disclosed and apparent to the person skilled in the art and are encompassed by the description and the Examples of the present invention. Further literature on the above-mentioned excipients as well as on electronic means that can be used in accordance with the present invention can be taken from the prior art, for example from public libraries using, e.g., electronic means. In addition, further public databases are readily available via the internet, like for example "PubMed".

Techniques for performing the present invention are known to the person skilled in the art and can be taken from the relevant literature, see for example Molecular Cloning A Laboratory Manual, 2nd Ed., ed. by Sambrook, Fritsch and Maniatis (Cold Spring Harbor Laboratory Press: 1989); Immunochemical Methods In Cell And Molecular Biology (Mayer and Walker, eds., Academic Press, London, 1987); Handbook Of Experimental Immunology, Volumes I-IV (D. M. Weir and C. C. Blackwell, eds., 1986).

### Brief description of the Figures

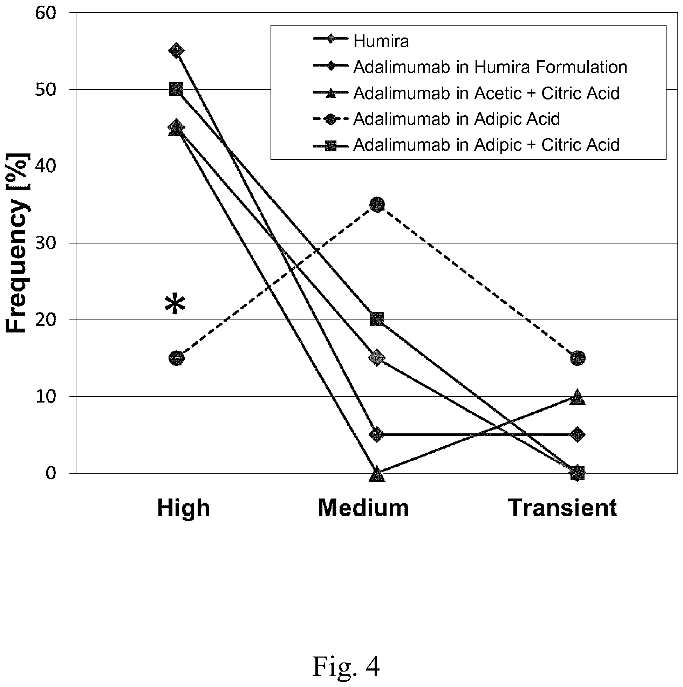

**Fig**. **1****-a:** Semi-log presentation of individual serum concentrations of adalimumab, following single s.c. administration of Humira^{®} (individuals with relevant ADA response marked by cycle)

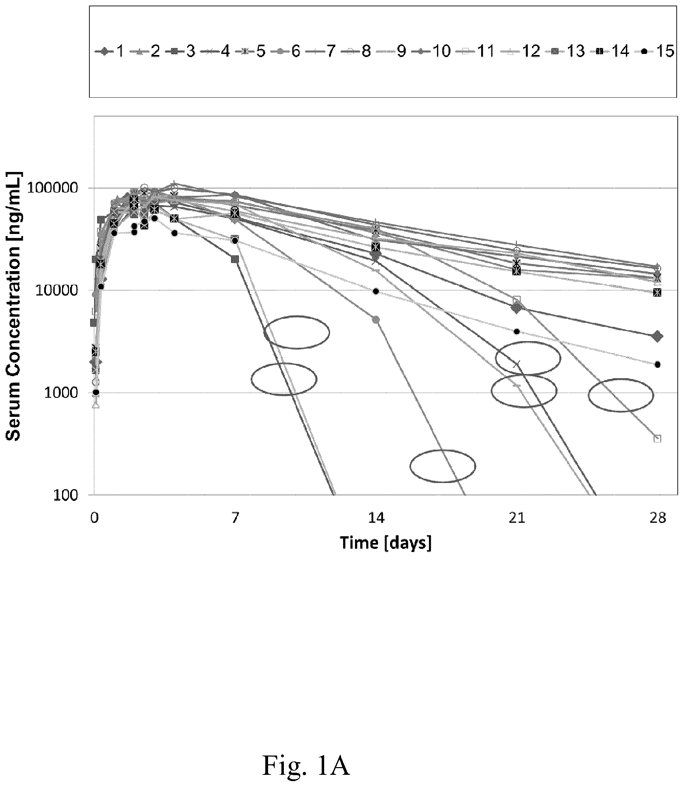

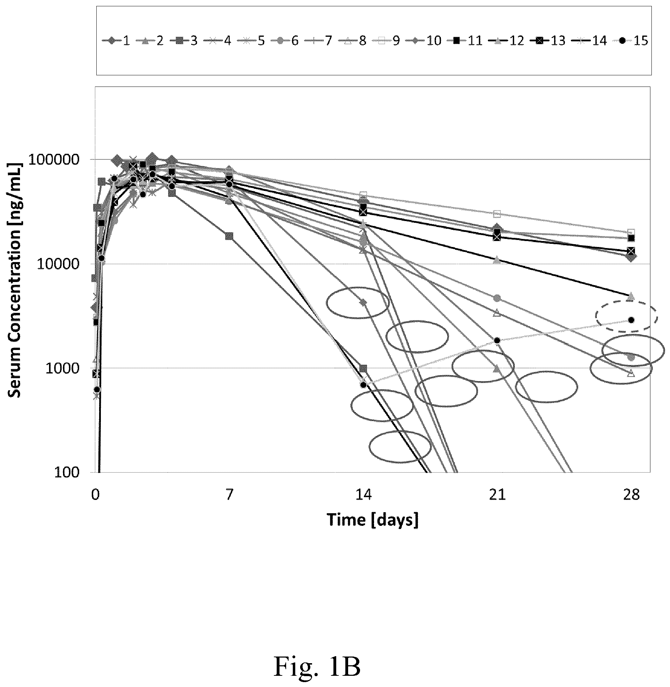

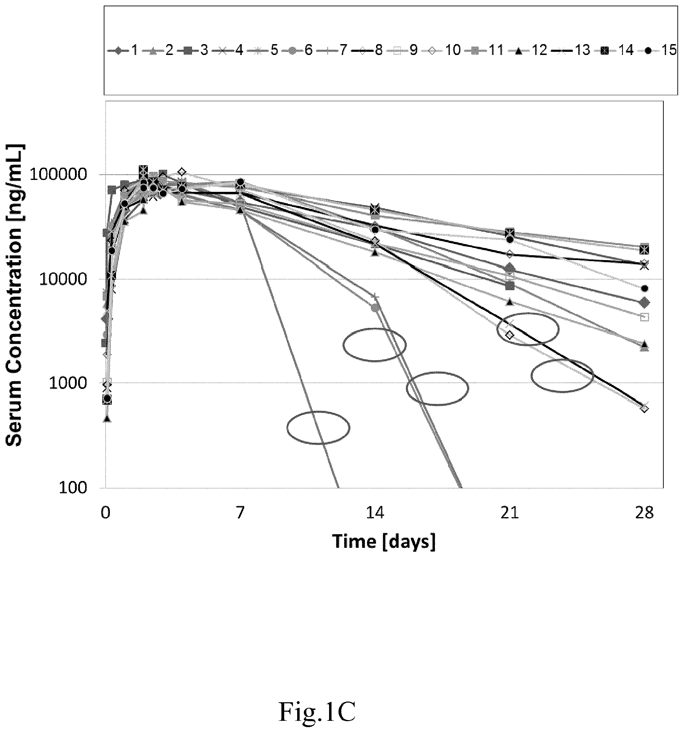

**Fig. 1****-b:** Semi-log presentation of individual serum concentrations of adalimumab, following single s.c. administration with assignee's adalimumab version, formulated like Humira^{®}, yet at an increased citric acid concentration (individuals with relevant ADA response are marked by cycle)
**Fig**. **1****-c:** Semi-log presentation of individual serum concentrations of adalimumab, following single s.c. administration with assignee's adalimumab version, formulated with adipic acid, and having a decreased citric acid concentration as compared to Humira^{®} (individuals with relevant ADA response are marked by cycle)
**Fig**. **2****:** Frequency of immunogenicity in study No 1. The correlation between the citric acid concentration and the rate of immunogenicity in the three groups of this study
**Fig**. **3****:** Frequency of high titre immune response in study No 2 for assignee's adalimumab version formulated the same way as Humira^{®}. The resulting immunogenicity is in the same order of magnitude, *i.e.* assignee's adalimumab version can be generalised to Humira^{®}, in this respect
**Fig. 4****:** Incidence of immunogenicity in study No 2. Immune response profile of the adipic acid formulation (high, medium, transient, no response) behaved statistically significantly as compared to Humira^{®} (p< 0,05 for Fisher Exact T test; Pearson: 9.167 (p= 0.02716) for Chi square, with 3 degrees of freedom and excluding cases of zero occurrence cell)

### EXAMPLES

### Material and Methods

### Example 1: Study No 1

### Animals:

45 purebred female New Zealand White (NZW) rabbits were supplied by a commercial breeder. Body weight was in the size order of approx. 3 kg and all animals were examined on external signs of illness to ensure inclusion of healthy individuals only. The individuals were marked by tattooed numbers.

### Application:

All animals were dosed on study day 1 through a subcutaneous bolus injection into the back region by one animal technician. The injection speed was about 15 seconds/dose at an administration volume of 0.2 mL/kg b.w. Treatment groups:

Three treatment groups were determined, two of which (groups 1 and 3) were treated with a formulation comprising assignee's adalimumab version, and one of which (group 2) comprising Humira^{®}.

**Table 1: Group size, Dose Level**

| **Group No** | **Treatment/ Formulation** | **Dose [mg/kg bw]** | **Application volume [mL/kg bw]** | **Number/ gender** |
|---|---|---|---|---|
| 1 | phosphate/citric acid | 10 | 0.2 | 15f |
| 2 | Humira^{®} | 10 | 0.2 | 15f |
| 3 | adipic/citric acid | 10 | 0.2 | 15f |

The animals are allocated to the groups by means of a computer generated randomization.

### Diet/Housing/Drinking Water:

A certified commercial feed (ssniff^{®} K-H (ssniffSpezialdiäten GmbH, 59494 Soest, Germany) and drinking tap water was provided to animals *ad libitum.* And the animals were housed individually in standard cages at a room temperature of about 20°C ± 3°C and a relative humidity of 55% ± 15%. Short term deviations occurred during cleaning phases. The housing rooms were lit (about 150 lux at approx. 1.50 m room height) on a 12-hour light/12-hour dark cycle.

### Blood sampling:

Blood samples were collected at the following time-points: pre-dose, 2, 8 (test day 1), 24, 40 (test day 2), 48, 60 h p.a. and on test days 4, 5, 8, 15, 22, 29. The sample was processed to serum and stored frozen at -20°C or colder until shipment and analysis of adalimumab concentration by a conventional sandwich ELISA, qualified for this purpose.

### Example 2: Study No 2

### Animals:

100 purebred male New Zealand White (NZW) rabbits were supplied by the same commercial breeder as in study No 1. A slightly increased group size, n=20/group vs. n=15/group in study No 1 was chosen to ensure increased robustness of the resulting data. Males were selected to assess whether the findings of study No 1 were not influenced by the gender. The animals' characteristics were the same as well, as were the remaining aspects of the study design, *i.e.* random assignment to study groups, dose level, application volume, diet, housing conditions, drinking water and light conditions, blood sample processing and sample storage.

### Treatment groups:

Five treatment groups were determined, four of which (groups 2 - 5) were treated with a formulation comprising assignee's adalimumab version, and one of which (group 1) comprising Humira^{®}.

**Table 2: Group size, Dose Level**

| **Group No** | **Treatment/ Formulation** | **Dose [mg/kg bw]** | **Application volume [mL/kg bw]** | **Number/ gender** |
|---|---|---|---|---|
| 1 | Humira^{®} | 10 | 0.2 | 20m |
| 2 | phosphate/citric acid | 10 | 0.2 | 20m |
| 3 | acetic/citric acid | 10 | 0.2 | 20m |
| 4 | adipic/citric acid | 10 | 0.2 | 20m |
| 5 | adipic acid | 10 | 0.2 | 20m |

### Blood sampling:

Blood sampling time-points for analysis of adalimumab serum concentrations and immunogenicity were slightly extended to ensure maximal clearance, minimising drug interference with the ADA detection. Samples collected at the following time-points: pre-dose, 2, 8 (test day 1), 24, 40 (test day 2), 56 h p.a. and on test days 4, 5 and at weekly intervals thereafter, *i.e.* test days 8, 15, 22, 29, 36, 43, 50, 57, 64, 71, 78. The sample was processed to serum and stored frozen at -20°C or colder until shipment and analysis of adalimumab concentration and ADA analysis by conventional ELISAs, validated for this purpose.

### Results

Figure 1-a illustrates the time course of adalimumab in study No 1, following a single s.c. injection. Starting about one week after dosing, a marked drop in serum levels occurred in about 40% of the individuals. Since target mediated drug disposition is ruled out in this species due to the high specificity of adalimumab to human and primate TNF, the increased clearance was very likely caused by immunogenicity, *i.e.* anti-drug antibodies, potentially with neutralising/inhibiting characteristics. Such increased clearance represents a key characteristic of antibody-drug complexes, as compared to the drug, to which no antibody is bound. Also the time-point of first occurrence, *i.e.* after one week, well matches with this interpretation, an effect to be expected for a human protein administered to an animal species, specifically if it was injected via a route at risk for provoking immunogenicity, such as subcutaneous administration.

Figure 1-b illustrates the outcome for the treatment of group 2 in this study, which was dosed with the same amount of assignee's adalimumab version, formulated as Humira^{®} yet using a slightly higher concentration of citric acid. Overall this change in formulation did not substantially impact the kinetics of resorption, *i.e.* the time-point when the maximal serum concentration was reached, nor the maximal concentration itself which both were in the same size order. Also in this second group a substantial fraction of animals showed the steep clearance indicating ADA formation, one week after dosing. Interestingly, even when disregarding one individual which showed only a transiently increased clearance, the fraction of animals developing an ADA response appeared higher as compared to group 1. While citric acid is known to cause a short lasting pain upon subcutaneous injection, a role of this frequently used component for immunogenicity of biopharmaceuticals is not described so far.

Group 3, was set-up to test whether the main buffer component of Humira^{®}, *i.e.,* phosphate, could be exchanged by adipic acid, without impacting key pharmacokinetic parameters and tolerability, which was the case. Adipic acid had been chosen as it favourably stabilises adalimumab against degradation and or aggregation processes. In order to ensure an unbiased comparison it was essential that the respective individuals were housed, handled and dosed the same way as groups 1 and 2. In addition to the exchange of phosphate by adipic acid, assignee's adalimumab version was administered at a citric acid concentration, decreased as compared to Humira^{®}, allowing to potentially minimise pain upon injection. Unexpectedly, not only the increased concentration of citric acid in group 2 resulted in an increased incidence of immunogenicity, but in addition, at a decreased citric acid concentration correlated with the lowest incidence of immunogenicity in this study (Figure 1-c).

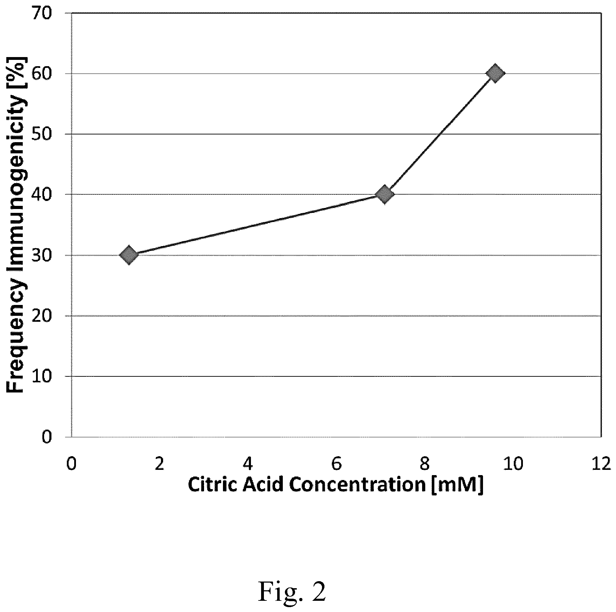

The correlation between the citric acid concentration and the rate of immunogenicity in the three groups of this study is summarised in Figure 2 and Table 3.

**Table 3: Overall ADA titer in study No 1**

| **Group No** | **Main Buffer component*** | **Concentration of main buffer component [mM]** | **Citric Acid [mM]** | **Incidence** | **Frequency** |
|---|---|---|---|---|---|
| 1 | Phosphate | 15.1 | **9.6** | 9/15 | 60% |
| 2** | Phosphate | 14.1 | **7.1** | 6/15 | 40% |
| 3 | Adipic Acid | 23 | **1.3** | 5/15 | 30% |
| *In addition, the buffers comprise following additional components [mg/mL]: NaCl 5.0-7.0; Mannitol 10.0-14.0; Tween80 0.5-1.5; NaOH q.s. to adjust pH to 5.20 | | | | | |
| **Marketed formulation of Humira^{®} | | | | | |

In order to further characterise and confirm the finding in study No 1 a second study was performed, with a design essentially reflecting the set-up of study No 1, yet the laboratory executing the in-life part of the study, *i.e.* animal housing, dosing and sample generation, was different. This step was taken to ensure that no uncontrolled factor may incidentally cause the observed effect of adipic acid / citric acid. As additional step a dedicated ADA assay had been developed, because this allows to directly demonstrate the modulation of the immune system, be it intended or unintended. Furthermore such an analysis allows to quantify the immune response, i. e. classify it to a high titre/neutralising/inhibiting, a medium or just a transient response.

Focusing on the clinically most critical high titre response, Figure 3 and Table 4 summarise the outcome of this study. First, it overall confirmed the key observation from the first study that the citric acid concentration strongly influences the immunogenicity of a drug recognised by the immune system, such as adalimumab.

Second, the data show that if assignee's adalimumab version is formulated the same way as Humira^{®}, the resulting immunogenicity is in the same order of magnitude, *i.e.* assignee's adalimumab version can be generalised to Humira^{®}, in this respect. This was underlined by the fact that also Cₘₐₓ, tₘₐₓ and AUC were equivalent as compared to Humira^{®}. At a higher concentration of citric acid, the immunogenicity of assignee's adalimumab version was lower as compared to a slightly lower citric acid concentration, but using adipic acid as main component. On the one hand, this shows again that citric acid very likely plays a role for immunogenicity over a diverse range of buffer systems. Figure 4 finally illustrates that the immune response profile of the adipic acid formulation (high, medium, transient, no response) behaved statistically significantly as compared to Humira^{®} (p< 0.05 for Fisher Exact T test; Pearson: 9.167 (p= 0.02716) for Chi square, with 3 degrees of freedom and excluding cases of zero occurrence cell). Without citric acid, the clinically relevant high titre response to the adipic acid formulation was negliably low.

Taken together these data show that the immune response can be modulated by selecting an appropriate concentration of the formulation or application solution and that, in addition, the use of adipic acid can result in a very low level of immune response. This can be specifically desirable for drugs, with the potential to be recognised by the immune system.

**Table 4: Occurrence of high titer responses in study No 2**

| **Group No** | **Main Buffer component*** | **Concentration of main buffer component [mM]** | **Citric Acid [mM]** | **Incidence** | **Frequency** |
|---|---|---|---|---|---|
| 1** | Phosphate | **14.1** | **7.1** | 9/20 | 45% |
| 2 | Phosphate | **14.1** | **7.1** | 11/20 | 55% |
| 3 | Acetic Acid | **20.3** | **2.4** | 9/20 | 45% |
| 4 | Adipic Acid | **23** | **1.4** | 10/20 | 50% |
| 5 | Adipic Acid | **23** | **-** | 3/20 | 15% |
| *In addition, the buffers comprise following additional components [mg/mL]: NaCl 5.0-7.0; Mannitol 10.0-14.0; Tween80 0.5-1.5; NaOH q.s. to adjust pH to 5.20 | | | | | |
| **Marketed formulation of Humira^{®} | | | | | |

## Claims

1. Use of hexanedioic acid, or at least one salt thereof, in the absence of citric acid for reducing the immunogenicity of a pharmaceutical formulation comprising an anti-TNFα antibody.

2. The use according to claim 1, wherein said use comprises dissolving said anti-TNFα antibody in a buffer comprising at least hexanedioic acid or at least one salt thereof.

3. The use according to any of the above claims, wherein the formulation further comprises at least one stabiliser selected from the group consisting of an amino acid, a sugar polyol, a disaccharide and/or a polysaccharide.

4. The use according to claim 3, wherein said disaccharide is at least one agent selected from the group consisting of sucrose, trehalose, maltose and/or lactose.

5. The use according to claim 3 or 4, wherein said sugar polyol is at least one agent selected from the group consisting of mannitol and/or sorbitol.

6. The use according to any one the above claims, wherein the formulation is designed to be administered intramuscularly or subcutaneously.

7. The use according to any one the above claims, wherein the formulation is designed to be administered non-orally.

## Patentansprüche

1. Verwendung von Adipinsäure, oder zumindest eines Salzes davon, in Abwesenheit von Zitronensäure zur Verringerung der Immunogenität einer pharmazeutischen Formulierung umfassend einen Anti-TNFα-Antikörper.

2. Die Verwendung gemäß Anspruch 1, wobei besagte Verwendung das Auflösen des besagten Anti-TNFα-Antikörpers in einem Puffer umfasst, der zumindest Adipinsäure, oder zumindest ein Salz davon, enthält.

3. Die Verwendung gemäß einem der oben genannten Ansprüche, wobei die Formulierung außerdem mindestens einen Stabilisator umfasst, ausgewählt aus der Gruppe bestehend aus einer Aminosäure, einem Zucker-Polyol, einem Disaccharid, und/oder einem Polysaccharid.

4. Die Verwendung gemäß Anspruch 3, wobei besagtes Disaccharid mindestens ein Agens ist, das ausgewählt ist aus der Gruppe bestehend aus Saccharose, Trehalose, Maltose und/oder Laktose.

5. Die Verwendung gemäß Anspruch 3 oder 4, wobei besagtes Zucker-Polyol mindestens ein Agens ist, das ausgewählt ist aus der Gruppe bestehend aus Mannitol und/oder Sorbitol.

6. Die Verwendung gemäß einem der oben genannten Ansprüche, wobei die Formulierung zur intramuskulären oder subkutanen Verabreichung hergerichtet ist.

7. Die Verwendung gemäß einem der oben genannten Ansprüche, wobei die Formulierung zur nicht-oralen Verabreichung hergerichtet ist.

## Revendications

1. Utilisation d'acide hexanedioïque, ou d'au moins un sel de ce dernier, en l'absence d'acide citrique pour réduire l'immunogénicité d'une formulation pharmaceutique comprenant un anticorps anti-TNFα.

2. Utilisation selon la revendication 1, dans laquelle ladite utilisation comprend la dissolution dudit anticorps anti-TNFα dans un tampon comprenant au moins de l'acide hexanedioïque ou au moins un sel de ce dernier.

3. Utilisation selon l'une quelconque des revendications précédentes, dans laquelle la formulation comprend en outre au moins un stabilisant sélectionné dans le groupe constitué par un acide aminé, un polyol de sucre, un disaccharide et/ou un polysaccharide.

4. Utilisation selon la revendication 3, dans laquelle ledit disaccharide est au moins un agent sélectionné dans le groupe constitué par le saccharose, le tréhalose, le maltose et/ou le lactose.

5. Utilisation selon la revendication 3 ou 4, dans laquelle ledit polyol de sucre est au moins un agent sélectionné dans le groupe constitué par le mannitol et/ou le sorbitol.

6. Utilisation selon l'une quelconque des revendications précédentes, dans laquelle la formulation est conçue pour être administrée de manière intramusculaire ou de manière sous-cutanée.

7. Utilisation selon l'une quelconque des revendications précédentes, dans laquelle la formulation est conçue pour être administrée de manière non orale.


### SEQUENCE LISTING

<110> Hexal AG
<120> Methods and formulations which allow the modulation of immune responses related to the administration of a biopharmaceutical drug
<130> LD41071
<160> 10
<170> PatentIn version 3.5
<210> 1
   <211> 1413
   <212> DNA
   <213> Artificial
<220>
   <223> Anti TNFalpha IgG Heavy chain Coding sequence
<400> 1
   atggcctggg tctggaccct gcctttcctg atggccgctg cccagtccgt gcaggccgag      60
   gtgcagctgg tcgagtctgg cggcggactg gtgcagcctg gccggtccct gcggctgtcc     120
   tgcgccgcct ccggcttcac cttcgacgac tacgccatgc actgggtccg ccaggcccct     180
   ggcaaaggcc tcgagtgggt gtccgccatc acctggaact ccggccacat cgactacgcc     240
   gactccgtgg agggccggtt caccatctcc cgggacaacg ccaagaactc cctgtacctg     300
   cagatgaact ccctgcgggc cgaggacacc gccgtgtact actgcgccaa ggtgtcctac     360
   ctgtccaccg cctcctccct ggactactgg ggccagggca ccctggtcac cgtgtcctcc     420
   gcctccacca agggcccctc cgtgttccct ctggcccctt cctccaagtc cacctccggc     480
   ggcaccgccg ctctgggctg cctggtcaag gactacttcc ctgagcctgt gacagtgtcc     540
   tggaactctg gcgccctgac cagcggcgtg cacaccttcc ctgccgtgct gcagtcctcc     600
   ggcctgtact ccctgtcctc cgtcgtcaca gtgccttcct ccagcctggg cacccagacc     660
   tacatctgca acgtgaacca caagccttcc aacaccaagg tggacaagaa ggtggagcct     720
   aagtcctgcg acaagaccca cacctgccct ccctgccctg cccctgagct gctgggcgga     780
   ccttccgtgt tcctgttccc tcctaagcct aaggacaccc tgatgatctc ccggacccct     840
   gaggtcacct gcgtggtggt ggacgtgtcc cacgaggatc ctgaggtcaa gttcaattgg     900
   tacgtggacg gcgtggaggt gcacaacgct aagaccaagc ctcgggaaga gcagtacaac     960
   tccacctacc gggtggtgtc cgtgctgacc gtgctgcacc aggactggct gaacggcaaa    1020
   gaatacaagt gcaaggtctc caacaaggcc ctgcctgccc ccatcgagaa aaccatctcc    1080
   aaggccaagg gccagcctcg cgagcctcag gtgtacaccc tgcctccctc ccgggacgag    1140
   ctgaccaaga accaggtgtc cctgacctgt ctggtcaagg gcttctaccc ttccgatatc    1200
   gccgtggagt gggagtccaa cggccagcct gagaacaact acaagaccac ccctcctgtg    1260
   ctggactccg acggctcctt cttcctgtac tccaagctga ccgtggacaa gtcccggtgg    1320
   cagcagggca acgtgttctc ctgctccgtg atgcacgagg ccctgcacaa ccactacacc    1380
   cagaagtccc tgtccctgag ccctggcaag tga                                 1413
<210> 2
   <211> 705
   <212> DNA
   <213> Artificial
<220>
   <223> Anti TNFalpha IgG Light chain Coding sequence
<400> 2
   atgtccgtgc tgacccaggt gctggccctg ctgctgctgt ggctgaccgg caccagatgc       60
   gacatccaga tgacccagtc cccctcctcc ctgtccgcct ccgtgggcga cagagtgacc      120
   atcacctgcc gggcctccca gggcatccgg aactacctgg cctggtatca gcagaagcct      180
   ggcaaggccc ctaagctgct gatctacgcc gcctccaccc tgcagtccgg cgtgccttcc      240
   cggttctccg gctccggcag cggcaccgac ttcaccctga ccatctcctc cctgcagcct      300
   gaggacgtgg ccacctacta ctgccagcgg tacaacagag ccccttacac cttcggccag      360
   ggcaccaagg tggagatcaa gcgtacggtg gccgctcctt ccgtgttcat cttccctccc      420
   tccgacgagc agctgaagtc cggcaccgcc agcgtcgtct gcctgctgaa caacttctac      480
   cctcgggagg ccaaggtgca gtggaaggtg gacaacgccc tgcagagcgg caactcccag      540
   gaatccgtca ccgagcagga ctccaaggac agcacctact ccctgtccag caccctgacc      600
   ctgtccaagg ccgactacga gaagcacaag gtgtacgcct gcgaggtcac ccaccagggc      660
   ctgtcctccc ccgtgaccaa gtccttcaac cggggcgagt gctga                     705
<210> 3
   <211> 470
   <212> PRT
   <213> Artificial
<220>
   <223> Anti TNFalpha IgG Heavy chain amino acid sequence
<400> 3
   Met Ala Trp Val Trp Thr Leu Pro Phe Leu Met Ala Ala Ala Gln Ser
   1               5                   10                  15
   Val Gln Ala Glu Val Gln Leu Val Glu Ser Gly Gly Gly Leu Val Gln
               20                  25                  30
   Pro Gly Arg Ser Leu Arg Leu Ser Cys Ala Ala Ser Gly Phe Thr Phe
           35                  40                  45
   Asp Asp Tyr Ala Met His Trp Val Arg Gln Ala Pro Gly Lys Gly Leu
       50                  55                  60
   Glu Trp Val Ser Ala Ile Thr Trp Asn Ser Gly His Ile Asp Tyr Ala
   65                  70                  75                  80
   Asp Ser Val Glu Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Val
               100                 105                 110
   Tyr Tyr Cys Ala Lys Val Ser Tyr Leu Ser Thr Ala Ser Ser Leu Asp
           115                 120                 125
   Tyr Trp Gly Gln Gly Thr Leu Val Thr Val Ser Ser Ala Ser Thr Lys
       130                 135                 140
   Gly Pro Ser Val Phe Pro Leu Ala Pro Ser Ser Lys Ser Thr Ser Gly
   145                 150                 155                 160
   Gly Thr Ala Ala Leu Gly Cys Leu Val Lys Asp Tyr Phe Pro Glu Pro
                   165                 170                 175
   Val Thr Val Ser Trp Asn Ser Gly Ala Leu Thr Ser Gly Val His Thr
               180                 185                 190
   Phe Pro Ala Val Leu Gln Ser Ser Gly Leu Tyr Ser Leu Ser Ser Val
           195                 200                 205
   Val Thr Val Pro Ser Ser Ser Leu Gly Thr Gln Thr Tyr Ile Cys Asn
       210                 215                 220
   Val Asn His Lys Pro Ser Asn Thr Lys Val Asp Lys Lys Val Glu Pro
   225                 230                 235                 240
   Lys Ser Cys Asp Lys Thr His Thr Cys Pro Pro Cys Pro Ala Pro Glu
                   245                 250                 255
   Leu Leu Gly Gly Pro Ser Val Phe Leu Phe Pro Pro Lys Pro Lys Asp
               260                 265                 270
   Thr Leu Met Ile Ser Arg Thr Pro Glu Val Thr Cys Val Val Val Asp
           275                 280                 285
   Val Ser His Glu Asp Pro Glu Val Lys Phe Asn Trp Tyr Val Asp Gly
       290                 295                 300
   Val Glu Val His Asn Ala Lys Thr Lys Pro Arg Glu Glu Gln Tyr Asn
   305                 310                 315                 320
   Ser Thr Tyr Arg Val Val Ser Val Leu Thr Val Leu His Gln Asp Trp
                   325                 330                 335
   Leu Asn Gly Lys Glu Tyr Lys Cys Lys Val Ser Asn Lys Ala Leu Pro
               340                 345                 350
   Ala Pro Ile Glu Lys Thr Ile Ser Lys Ala Lys Gly Gln Pro Arg Glu
           355                 360                 365
   Pro Gln Val Tyr Thr Leu Pro Pro Ser Arg Asp Glu Leu Thr Lys Asn
       370                 375                 380
   Gln Val Ser Leu Thr Cys Leu Val Lys Gly Phe Tyr Pro Ser Asp Ile
   385                 390                 395                 400
   Ala Val Glu Trp Glu Ser Asn Gly Gln Pro Glu Asn Asn Tyr Lys Thr
                   405                 410                 415
   Thr Pro Pro Val Leu Asp Ser Asp Gly Ser Phe Phe Leu Tyr Ser Lys
               420                 425                 430
   Leu Thr Val Asp Lys Ser Arg Trp Gln Gln Gly Asn Val Phe Ser Cys
           435                 440                 445
   Ser Val Met His Glu Ala Leu His Asn His Tyr Thr Gln Lys Ser Leu
       450                 455                 460
   Ser Leu Ser Pro Gly Lys
   465                 470
<210> 4
   <211> 234
   <212> PRT
   <213> Artificial
<220>
   <223> Anti TNFalpha IgG Light chain amino acid sequence
<400> 4
   Met Ser Val Leu Thr Gln Val Leu Ala Leu Leu Leu Leu Trp Leu Thr
   1               5                   10                  15
   Gly Thr Arg Cys Asp Ile Gln Met Thr Gln Ser Pro Ser Ser Leu Ser
               20                  25                  30
   Ala Ser Val Gly Asp Arg Val Thr Ile Thr Cys Arg Ala Ser Gln Gly
           35                  40                  45
   Ile Arg Asn Tyr Leu Ala Trp Tyr Gln Gln Lys Pro Gly Lys Ala Pro
       50                  55                  60
   Lys Leu Leu Ile Tyr Ala Ala Ser Thr Leu Gln Ser Gly Val Pro Ser
   65                  70                  75                  80
   Arg Phe Ser Gly Ser Gly Ser Gly Thr Asp Phe Thr Leu Thr Ile Ser
                   85                  90                  95
   Ser Leu Gln Pro Glu Asp Val Ala Thr Tyr Tyr Cys Gln Arg Tyr Asn
               100                 105                 110
   Arg Ala Pro Tyr Thr Phe Gly Gln Gly Thr Lys Val Glu Ile Lys Arg
           115                 120                 125
   Thr Val Ala Ala Pro Ser Val Phe Ile Phe Pro Pro Ser Asp Glu Gln
       130                 135                 140
   Leu Lys Ser Gly Thr Ala Ser Val Val Cys Leu Leu Asn Asn Phe Tyr
   145                 150                 155                 160
   Pro Arg Glu Ala Lys Val Gln Trp Lys Val Asp Asn Ala Leu Gln Ser
                   165                 170                 175
   Gly Asn Ser Gln Glu Ser Val Thr Glu Gln Asp Ser Lys Asp Ser Thr
               180                 185                 190
   Tyr Ser Leu Ser Ser Thr Leu Thr Leu Ser Lys Ala Asp Tyr Glu Lys
           195                 200                 205
   His Lys Val Tyr Ala Cys Glu Val Thr His Gln Gly Leu Ser Ser Pro
       210                 215                 220
   Val Thr Lys Ser Phe Asn Arg Gly Glu Cys
   225                 230
<210> 5
   <211> 9
   <212> PRT
   <213> Artificial
<220>
   <223> Anti TNFalpha IgG Light chain CDR 3 amino acid sequence
<220>
   <221> misc_feature
   <222> (9)..(9)
   <223> Xaa can be any naturally occurring amino acid
<400> 5
   Gln Arg Tyr Asn Arg Ala Pro Tyr Xaa
   1               5
<210> 6
   <211> 12
   <212> PRT
   <213> Artificial
<220>
   <223> Anti TNFalpha IgG Heavy chain CDR 3 amino acid sequence
<220>
   <221> misc_feature
   <222> (12)..(12)
   <223> Xaa can be any naturally occurring amino acid
<400> 6
   Val Ser Tyr Leu Ser Thr Ala Ser Ser Leu Asp Xaa
   1               5                   10
<210> 7
   <211> 7
   <212> PRT
   <213> Artificial
<220>
   <223> Anti TNFalpha IgG Light chain CDR 2 amino acid sequence
<400> 7
   Ala Ala Ser Thr Leu Gln Ser
   1               5
<210> 8
   <211> 17
   <212> PRT
   <213> Artificial
<220>
   <223> Anti TNFalpha IgG Heavy chain CDR 2 amino acid sequence
<400> 8
   Ala Ile Thr Trp Asn Ser Gly His Ile Asp Tyr Ala Asp Ser Val Glu
   1               5                   10                  15
   Gly
<210> 9
   <211> 11
   <212> PRT
   <213> Artificial
<220>
   <223> Anti TNFalpha IgG Light chain CDR 1 amino acid sequence
<400> 9
   Arg Ala Ser Gln Gly Ile Arg Asn Tyr Leu Ala
   1               5                   10
<210> 10
   <211> 5
   <212> PRT
   <213> Artificial
<220>
   <223> Anti TNFalpha IgG Heavy chain CDR 1 amino acid sequence
<400> 10
   Asp Tyr Ala Met His
   1               5